# EUROPEAN PATENT APPLICATION

(11) **EP 2 614 799 A1**
(43) Date of publication of application: **17.07.2013**
(21) Application number: 13150113.2
(22) Date of filing: 03.01.2013
(51) Int. Cl.: A61F 7/02, A61F 7/00

(54) **Body temperature maintaining apparatus**

(30) Priority: 10.01.2012 JP 2012001793
(71) Applicant: Nihon Kohden Corporation, Shinjuku-ku Tokyo (JP)
(72) Inventor: Takayanagi, Yoshihiro, Tokyo (JP)
(74) Representative: Ilgart, Jean-Christophe

(57) **Abstract**

A body temperature maintaining apparatus is used on occasion of emergent transportation, utilizing an air conditioner of an emergent transportation vehicle or the like. The body temperature maintaining apparatus includes a heat receiving part for receiving heat of an air blown from an air conditioner mounted on a mobile body for carrying a patient, a heat applying part attached to a living body part of the patient thereby to apply the heat, a tube body which interconnects the heat receiving part and the heat applying part for feeding a heat medium from the heat receiving part to the heat applying part, and a forcibly moving unit provided in at least one of the heat receiving part, the heat applying part, and the tube body, and adapted to forcibly move the heat medium from the heat receiving part to the heat applying part.

## Description

### BACKGROUND OF THE INVENTION

The present invention relates to a body temperature maintaining apparatus which can be used on occasion of emergent transportation.

Conventionally, a low temperature curing instrument and a body temperature maintaining apparatus have been clinically used, and a remarkably large-sized apparatus or system has been heretofore proposed. In this connection, it is important to start low temperature treatment as early as possible, on occasion of a stroke or cardiopulmonary resuscitation, and this has come to particular attention of a medical society.

However, the body temperature maintaining apparatus which is proposed at present is the apparatus to be used in facilities such as a hospital. Therefore, it is impossible to mount the existing body temperature maintaining apparatus in a limited space, for example, in an ambulance or a helicopter on occasion of emergent transportation, which hinders the low temperature treatment from being conducted at an early time.

Under the circumstances, it is considered to utilize an air conditioning equipment in an emergent transportation vehicle or the like. However, such a system that an airflow blown from an air conditioner or the like is applied to a heat exchanger thereby to conduct heat exchange, and cooling or warming action is performed by feeding heat medium to a desired position through a hose, as disclosed in Patent Document 1, has been only considered. This system does not yet satisfy a requirement for an apparatus to be mounted on the emergent transportation vehicle or the like in which it is required to rapidly and efficiently cool or warm body temperature of a patient so that a desired part of the patient may be cooled or warmed. Particularly, because the patient is unconscious in some cases, there remains such possibility that the patient is too much cooled or too much warmed, although delicate attention must be paid to management of the body temperature of the patient.

(Patent Document 1) Japanese Patent Publication No. JP-A-H11-197173

### SUMMARY

This invention provides a body temperature maintaining apparatus which can be used on occasion of the emergent transportation, by utilizing an air conditioning equipment in an emergent transportation vehicle, an airplane, etc.

It is therefore an aspect of the invention to provide a body temperature maintaining apparatus including
a heat receiving part configured to receive heat of an air blown from an air conditioner which is mounted on a mobile body for carrying a patient,
a heat applying part which is attached to a living body part of the patient thereby to apply the heat,
a tube body which interconnects the heat receiving part and the heat applying part for feeding a heat medium from the heat receiving part to the heat applying part, and
a forcibly moving unit which is provided in at least one of the heat receiving part, the heat applying part, and the tube body, and adapted to forcibly move the heat medium from the heat receiving part to the heat applying part.

The body temperature maintaining apparatus may further include
a temperature sensor configured to detect temperature of the heat applying part and/or temperature of the patient,
a temperature setting unit configured to set the temperature, and
a temperature controlling part configured to control the forcibly moving unit according to the temperature which is detected by the temperature sensor and the temperature which has been set by the temperature setting unit.

The heat receiving part may be provided with an attaching and detaching unit for attaching and detaching the heat receiving part to and from the air conditioner which is mounted on the mobile body.

The heat applying part may include
a heat source part in which the tube body is folded back in a zigzag shape,
a surface part which is provided at a side close to the living body part from the heat source part, and has an adhesive agent formed on its surface and adapted to be adhered to the living body,
a heat insulating material which is provided at an opposite side to the living body part from the heat source part thereby to prevent the heat of the heat source part from being radiated to an exterior, and
a back face part which is connected to the surface part, thereby to interpose the heat source part and the heat insulating material between the surface part and the back face part.

The temperature controlling part may be configured to control the forcibly moving unit so as to keep the temperature substantially constant.

The heat medium may be circulated in the heat receiving part, the tube body, and the heat applying part.

The heat applying part may be directly attached to at least one of a neck, a breast, and a thigh of the patient.

The heat applying part may be in a shape of a blanket.

The heat medium may be an air or liquid.

### BRIEF DESCRIPTION OF THE DRAWINGS

Fig. 1 is a block diagram of a body temperature maintaining apparatus in an embodiment according to the invention.
Fig. 2 is a perspective view showing an internal structure of an essential part of the body temperature maintaining apparatus in the embodiment according to the invention.
Fig. 3 is a view showing a manner of using the body temperature maintaining apparatus in the embodiment according to the invention.
Fig. 4 is a sectional view taken along a line A-A in Fig. 2.

### DETAILED DESCRIPTION OF EMBODIMENTS

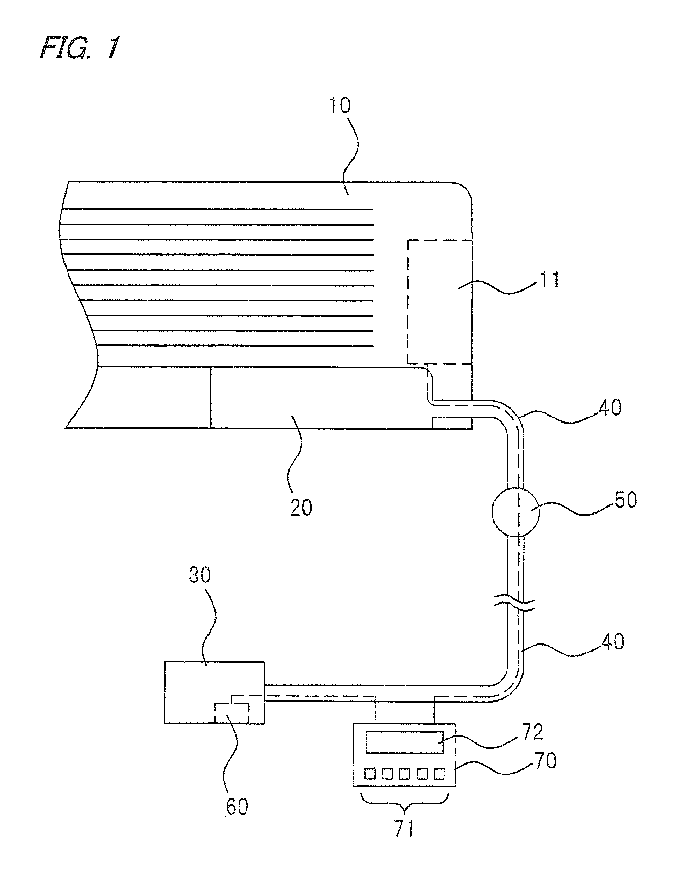

Now, a body temperature maintaining apparatus in an embodiment according to the invention will be described referring to the attached drawings. In the respective drawings, the same constituent elements are denoted with the same reference numerals, and overlapped descriptions are omitted. As shown in Fig. 1, the body temperature maintaining apparatus in the embodiment according to the invention is attached to an air conditioner 10 which is mounted on a mobile body for carrying a patient. Herein, the mobile body includes an automobile such as an ambulance, and further, a helicopter, a small airplane, and a ship.

The body temperature maintaining apparatus in the embodiment is provided with a heat receiving part 20 which receives heat of an air blown from the air conditioner 10, a heat applying part 30, a tube body 40, and a forcibly moving unit 50. The heat applying part 30 is attached to a living body part of a patient thereby to apply the heat to the living body part. The tube body 40 interconnects the heat receiving part 20 and the heat applying part 30, for the purpose of feeding a heat medium from the heat receiving part 20 to the heat applying part 30. The heat receiving part 20 may be provided with an attaching and detaching unit such as an engaging hook for enabling the heat receiving part to be attached to or detached from the air conditioner 10.

In case where the heat medium is the air, the heat receiving part 20 may be provided with an opening for taking the air which is blown from the air conditioner 10, as it is. In case where the heat medium is liquid such as water, the heat receiving part 20 may only be a casing which is engaged with an air blowing port of the air conditioner 10. In this case, the casing may be provided with fins or the like for securing a large contact area with the heat medium, thereby to obtain a favorable heat transmission. In case where the heat medium is the liquid such as water, the apparatus is so constructed as to circulate the heat medium between the heat receiving part 20 and the heat applying part 30 by way of the tube body 40. The forcibly moving unit 50 is a unit for forcibly moving the heat medium from the heat receiving part 20 to the heat applying part 30, and can include a fan, a pump and a valve. The forcibly moving unit 50 may be provided in at least one of the heat receiving part 20, the heat applying part 30, and the tube body 40.

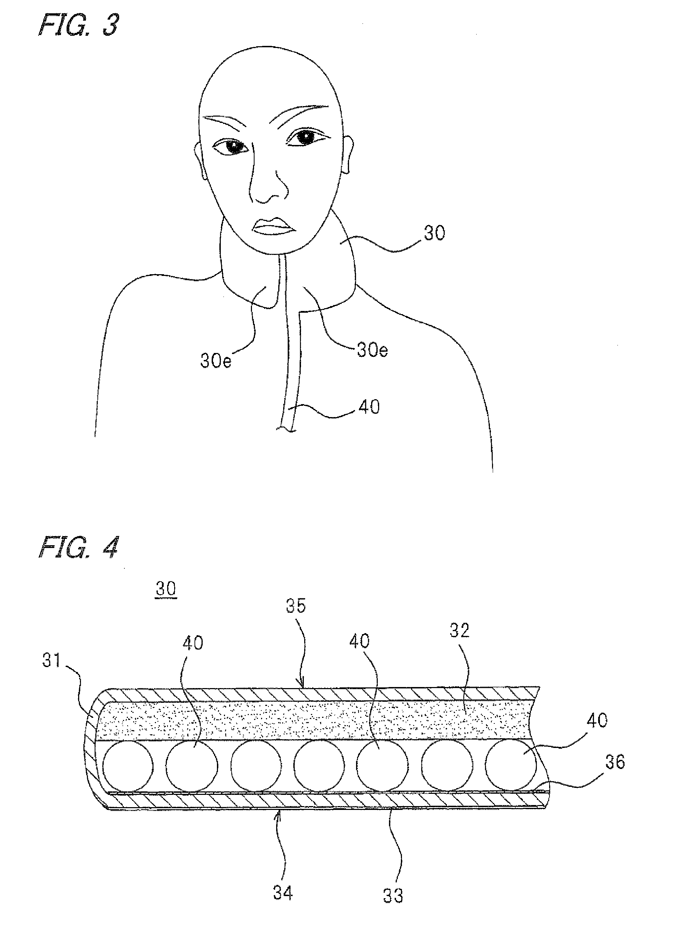

The heat applying part 30 is attached to the living body part of the patient. Although the living body part is not limited, it is desirable to attach the heat applying part 30 to a neck portion, a breast or a thigh where a thick blood vessel exists relatively near a surface of the body, thereby to lower or to raise the body temperature. In Fig. 3, there is shown a perspective view of the embodiment in which the heat applying part 30 is attached to the neck portion. As apparent from the drawing, the heat applying part 30 is formed by rounding a sheet of plate-like material into a cylindrical shape in a manner of surrounding the neck portion substantially circumferentially, and so shaped as to be elastically rounded as a whole, and to be fitted to the neck portion. Therefore, on occasion of attaching the heat applying part 30, it is possible to attach the heat applying part 30 in a manner of wrapping the neck of the patient from a back side, by gripping, for example, end portions 30e, 30e at both sides of a slit which is formed at a position of a throat, and by spreading the cylindrical shape into a flat plate shape. Of course, a shape of the heat applying part 30 may be appropriately modified according to the living body part where the apparatus is to be used. It is to be noted that the heat applying part 30 is not limited to the above described shape, but may be, for example, in a form of a blanket for covering the body of the patient.

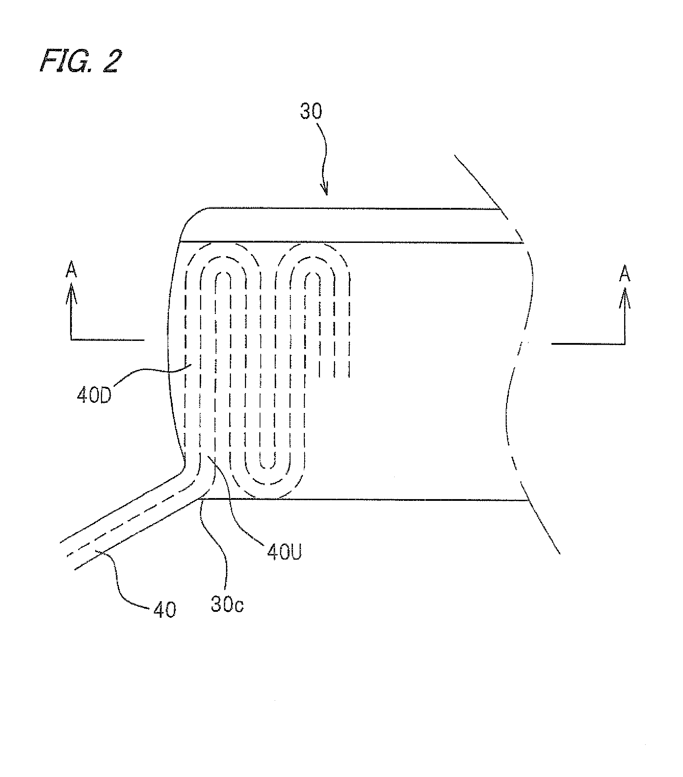

As apparent from Fig. 2 showing the heat applying part 30 in a state developed in a flat plate shape, the tube body 40 is extended to an interior of the heat applying part 30 from its corner part 30c. The embodiment shown in Fig. 2 uses the liquid as the heat medium. The tube body 40 includes a downstream side tube body 40D which is extended downward from the heat receiving part 20 to the heat applying part 30, and an upstream side tube body 40U which is folded back at an end of the heat applying part 30 where the corner part 30c is not provided, then, reaches the corner part 30c, and thereafter, is extended from the heat applying part 30 to the heat receiving part 20.

The heat applying part 30 is so constructed that an outer covering 31 formed of, for example, a thick cloth, as a whole, covers an entirety of the heat applying part 30. Inside the outer covering 31, the tube body 40 (40D, 40U) as a heat source is folded in a zigzag shape and arranged in parallel, and a heat insulating material 32 for preventing the heat of the tube body 40 from being radiated to an exterior is provided at an opposite side to the living body part.

As shown in Fig. 4, the outer covering 31 at a side of the living body part forms a surface part 34 which has a layer of adhesive agent 33 on a surface thereof to be adhered to the living body. A protecting seal such as paper is attached to the layer of the adhesive agent 33 in a state where the apparatus is not in use. The outer covering 31 at the opposite side to the living body part forms a back face part 35 to be connected to the surface part 34. The tube body 40 and the heat insulating material 32 are interposed between the surface part 34 and the back face part 35. Moreover, a heat conductive material 36 such as an aluminum foil in a shape of sheet is interposed between the surface part 34 and the tube body 40.

Further, in this embodiment, a temperature sensor 60 is provided in the heat applying part 30, and a controller 70 is equipped at an appropriate position. The controller 70 is provided with several switches, an operating part 71 which is a temperature setting unit for setting the temperature, and a display part 72 for displaying the temperature which has been set, current temperature, and so on. Processes of the controller 70 are performed by a CPU. Although not shown, the temperature sensor 60 may be, of course, in such a shape as to be directly attached on the patient for measuring the body temperature of the patient. In this case, the body temperature of the patient is directly measured, and therefore, it is possible to efficiently manage the body temperature of the patient, without cooling or warming the patient too much. Moreover, the temperature sensor 60 may be so arranged as to detect both the temperature of the heat applying part 30 and the temperature of the patient.

A detection signal of the temperature sensor 60 is transmitted to the controller 70. The controller 70 is connected to a control board 11 of the air conditioner 10, and functions as a temperature control part for controlling the forcibly moving unit 50, by supplying control signals for switching on or off the power supply, for conducting conversion between cooling and warming, and for controlling the temperature thereby to control the temperature of the airflow. Further, the controller 70 may be so constructed as to supply a control signal for changing a moving speed of the heat medium, to the forcibly moving unit 50. A power supply of the controller 70 may be obtained from the air conditioner 10 or may be obtained from a separate power source, although not shown.

The body temperature maintaining apparatus having the above described structure can be used in the following manner. When the heat receiving part 20 which can be attached or detached is in a detached state, mounting of the apparatus is performed by attaching the heat receiving part 20 to a predetermined position of the air conditioner 10. Then, a predetermined switch of the operating part 71 of the controller 70 is operated to apply the power supply thereby to set the required temperature and the moving speed of the heat medium. Thereafter (of course, may be prior to that), the heat applying part 30 is attached to a desired position of the patient. In this case, the protecting seal such as paper is peeled off, so that the layer of the adhesive agent 33 on the surface part 34 may be firmly adhered to a required position of the living body (a carotid part, in case of the neck), for enabling the heat to be adequately transmitted.

The controller 70 controls the control board 11 and the forcibly moving unit 50 according to the temperature which have been set and the moving speed of the heat medium. Moreover, the controller 70 receives the detection signal from the temperature sensor 60, and controls the control board 11 according to a difference from the temperature which has been set, and continues to control so that the heat applying part 30 may be kept at the temperature which has been set.

According to the body temperature maintaining apparatus of the invention, because the apparatus is provided with the heat receiving part for receiving heat of an air blown from an air conditioner which is mounted on a mobile body for carrying a patient, it is possible to rapidly and efficiently control body temperature of a patient during emergent transportation, without requiring a particular space. Moreover, because the heat applying part for applying the heat to the living body part is so constructed as to be attached to the living body part of the patient, it is possible to easily attach the heat applying part to the patient who must be urgently carried.

According to the body temperature maintaining apparatus of the invention, because the temperature of the air blown from the air conditioner is controlled according to the temperature which is detected by the temperature sensor and the temperature which is set by the temperature setting unit, it is possible to control the temperature of the air at a required temperature thereby to maintain the body temperature of the patient. Particularly, even in case where the patient is unconscious, the patient will not be cooled or warmed too much. Moreover, because the heat receiving part is provided with the attaching and detaching unit for attaching and detaching the heat receiving part to and from the air conditioner which is mounted on the mobile body, it is possible to attach or detach the heat applying part in case of necessity.

According to the body temperature maintaining apparatus of the invention, the adhesive agent which is adapted to be adhered to the living body is provided on the surface of the heat applying part, it is possible to adequately attach the heat applying part to a required position of the living body thereby to control the body temperature. Moreover, because the heat insulating material for preventing the heat of the heat source part from being radiated to an exterior is provided at the opposite side to the living body part, it is possible to efficiently control the body temperature.

## Claims

1. A body temperature maintaining apparatus comprising
a heat receiving part configured to receive heat of an air blown from an air conditioner which is mounted on a mobile body for carrying a patient,
a heat applying part which is attached to a living body part of the patient thereby to apply the heat,
a tube body which interconnects the heat receiving part and the heat applying part for feeding a heat medium from the heat receiving part to the heat applying part, and
a forcibly moving unit which is provided in at least one of the heat receiving part, the heat applying part, and the tube body, and adapted to forcibly move the heat medium from the heat receiving part to the heat applying part.

2. A body temperature maintaining apparatus as claimed in claim 1, further including
a temperature sensor configured to detect temperature of the heat applying part and/or temperature of the patient,
a temperature setting unit configured to set the temperature, and
a temperature controlling part configured to control the forcibly moving unit according to the temperature which is detected by the temperature sensor and the temperature which has been set by the temperature setting unit.

3. A body temperature maintaining apparatus as claimed in claim 1 or 2, wherein the heat receiving part is provided with an attaching and detaching unit for attaching and detaching the heat receiving part to and from the air conditioner which is mounted on the mobile body.

4. A body temperature maintaining apparatus as claimed in any one of claims 1 to 3, wherein the heat applying part includes
a heat source part in which the tube body is folded back in a zigzag shape,
a surface part which is provided at a side close to the living body part from the heat source part, and has an adhesive agent formed on its surface and adapted to be adhered to the living body,
a heat insulating material which is provided at an opposite side to the living body part from the heat source part thereby to prevent the heat of the heat source part from being radiated to an exterior, and
a back face part which is connected to the surface part, thereby to interpose the heat source part and the heat insulating material between the surface part and the back face part.

5. A body temperature maintaining apparatus as claimed in any one of claims 2 to 4, wherein the temperature controlling part is configured to control the forcibly moving unit so as to keep the temperature substantially constant.

6. A body temperature maintaining apparatus as claimed in any one of claims 1 to 5, wherein the heat medium is circulated in the heat receiving part, the tube body, and the heat applying part.

7. A body temperature maintaining apparatus as claimed in any one of claims 1 to 6, wherein the heat applying part is directly attached to at least one of a neck, a breast, and a thigh of the patient.

8. A body temperature maintaining apparatus as claimed in any one of claims 1 to 6, wherein the heat applying part is in a shape of a blanket.

9. A body temperature maintaining apparatus as claimed in any one of claims 1 to 8, wherein the heat medium is an air or liquid.
